Europäisches Patentamt

European Patent Office

Office européen des brevets

(19)

(11) Publication number: 0 312 220
A1

# (12) EUROPEAN PATENT APPLICATION

(21) Application number: 88308957.5

(22) Date of filing: 27.09.88

(51) Int. Cl.4: **A23L 1/105 , A23L 1/30 , C13K 11/00 , C12P 19/24**

(30) Priority: 28.09.87 US 101564
28.09.87 US 101561

(43) Date of publication of application:
19.04.89 Bulletin 89/16

(84) Designated Contracting States:
AT BE CH DE ES FR GB GR IT LI LU NL SE

(71) Applicant: **NABISCO/CETUS FOOD BIOTECHNOLOGY RESEARCH PARTNERSHIP**
1400 Fifty-Third Street
Emeryville California 94608(US)

(84) **BE CH DE FR GB LI LU NL SE AT**

(71) Applicant: **NABISCO BRANDS, INC.**
100 DeForest Avenue
East Hanover New Jersey 07936(US)

(84) **ES GR IT**

(72) Inventor: **Maselli, John A.,**
529, Knobview Place,
Winston-Salem, North Carolina 21704,(US)
Inventor: **Neidleman, Saul L.,**
5377, Hilltop Crescent,
Oakland, California 94618,(US)
Inventor: **Antrim, Richard L.,**
80, Warren Road,
Sparta, New Jersey 07871,(US)
Inventor: **Johnson, Richard A.,**
403, North 10th Street,
Clinton, Iowa 52732,(US)

(74) Representative: **Crisp, David Norman et al**
D. YOUNG & CO. 10 Staple Inn
London, WC1V 7RD(GB)

(54) **Cereal products naturally sweetened with fructose.**

(57) Breakfast cereals are sweetened by treating cereal grains or at least one cereal grain fraction such as bran, with enzymes comprising glucose isomerase to produce fructose while retaining cereal particle discreteness or integrity. A portion of the starch and/or cellulose of the cereal grains or at least one grain fraction is enzymatically converted to dextrose or glucose which is converted to fructose by the glucose isomerase. Enzymes which may be used for the cellulose and/or starch conversion include cellulases, alpha-amylases, and glucoamylases. Enzymatic treatment with alpha-amylase may be initiated prior to, during, or after cooking. The enzymatically treated, cooked cereal grains are formed into breakfast cereal shapes and the enzymes are inactivated to provide a shelf-stable cereal product. The cereal products exhibit a sweet, pleasing complex honey-like taste and aroma. Producing fructose provides a greater level of sweetness for a given amount of starch conversion into low molecular weight reducing sugars such as mono- and di-saccharides. In achieving a given level of sweetness, more starch or high molecular weight dextrins may be retained for their matrix forming

ability or for improved machinability of the enzymatically treated cereal grains into breakfast cereal shapes. The naturally sweetened cereal products of the present invention may be in shredded, flaked, ground, or extruded form.

# CEREAL PRODUCTS NATURALLY SWEETENED WITH FRUCTOSE

## FIELD OF THE INVENTION

This invention relates to the production of breakfast cereals by the treatment of cereal grains or cereal grain fractions with enzymes.

## BACKGROUND OF THE INVENTION

Grains provide a rich source of proteins and complex carbohydrates, both of which are necessary ingredients for a well-balanced diet. In addition, grains may be deformed from their discrete shapes into a multitude of forms including flakes, shreds, flours and the like. Starch, composed of both amylose and amylopectin provides for formability of grains into ready-to-eat breakfast cereals, hot cereals, breads, and other baked goods.

Bran, a cereal grain fraction, is relatively low in starch. In the process of U.S. Patent No. 4,500,558 to Fulger et al, bran is modified by extrusion so that it becomes more readily millable. According to U.S. Patent No. 4,500,558, from 10 to 25% starch is naturally present or is added to the bran material, the starch functioning as a vapor-lock to build pressure and to coat the bran during extrusion.

Milled grains, cooked or otherwise, generally have a bland and undifferentiated taste. Many schemes have been conceived and protocols designed to treat cereal grains and render their taste sweeter, more complex and more differentiated.

The conversion of grain starches into smaller components has a long history. Hydrolysis of the long polysaccharide chains into shorter chains and monomers such as glucose and maltose may be performed by treating starches with dilute acids, dilute alkalis or by enzymatic catalyzed reactions.

Although there is some evidence for xylose isomerase activity in wheat germ, (Pubols, M. H., et al., Plant Physiology 38, 454 [1962]) and other higher plants (Bartfay, J., Nature 185, 924 [1960]), still, the enzyme would be expected to be denatured during cooking.

Formability and/or product breakage problems tend to arise where grain is altered such that a sufficient amount of maltose or glucose i.e., dextrose, is produced in sweetening amounts for breakfast cereals. Starch is generally needed for its matrix forming abilities so the grain may be deformed, blended, and conformed into ready-to-eat end products such as shredded wheat, cereal flakes, and expanded or puffed cereals.

Conversion of fiber in cereals to different forms has received less, but significant, attention in the past because the major portion of fiber can be removed from grains in the grain milling process. In those processes known in the art in which the cellulose content of the cereal grain is treated, the modifications are primarily directed to improving the accessibility of the starch fraction of the grain for further processing or for recovery of food value from a removed fiber fraction.

In U.S. Patent No. 4,656,040 to Fulger et al a matrix forming ingredient which is either a modified bran fraction, a toasted ground germ fraction, or combination thereof is admixed with an enzymatically hydrolyzed endosperm fraction. The endosperm fraction, it is disclosed, contains approximately 95% of the starch of the whole grain and treating it alone, with alpha-amylase and glucoamylase, avoids off-flavor development.

Treatment of cereal grains and subcomponents therein with proteolytic enzymes is disclosed in U.S. Patents Nos.: 1,178,039 to Wahl; 2,853,388 to Kiely; 3,157,513 to Allen et al.; 3,243,301 to Hesseltine et al.; 4,056,637 to Hagiwara et al. Japanese examples of such treatments are taught in: Japanese Patent Publication No. 53-62848, published June 5, 1978; and Japanese Patent Publication No. 57-47465, published March 18, 1982. However, none of these cited references teach the production of shredded, flaked, or extruded cereals.

The production of cereal products which may be in shredded, flaked or extruded form by treatment of cereal grains with proteolytic enzymes is taught in U.S. Patents Nos.: 976,332 to Anhaltzer; 1,541,263 to Hoffman et al.; 3,664,848 to Bedenk et al.; and 4,282,319 and 4,377,602 to Conrad.

In these processes for producing cereal products using proteolytic enzymes, a whole cereal grain or a bran fraction is not treated enzymatically so as to retain starch or high molecular weight dextrins for their matrix forming ability.

The enzymatic treatment of cereal grains using cellulase or a combination of alpha amylase, amyloglucosidase, and glucose isomerase are disclosed in U.S. Patents Nos.: 4,069,103 to Muller; 4,089,745 to Antrim et al.; 4,247,636 to Schoenrock et al.; 4,292,331 to Ostre; 4,378,432 to Castelli et al.; 4,458,017 to Horvath et al.; and 4,501,814 to Schoenrock et al. Processes using starch attacking enzymes are also taught in European Patent Application No. 78,782 and Swiss Patent Publication No. 622,028, published March 13, 1981. Japanese Publication 57-47363 teaches the production of a cereal tea by treating cereal grains with heat between 100-200°C to puff the cereal, followed by treatment with an enzyme such as cellulase. The enzymatically treated cereal is heat dried and then roasted.

However, the products resulting from these enzymatic treatments with cellulase or alpha amylase, glucoamylase, and glucose isomerase are not cereals. Retention of starch for its matrix forming properties so as to provide formability of the enzymatically treated cereals is not taught in these references.

Enzymatic treatment of ground grains or grain fractions with sugar-producing enzymes in the preparation of cereal products is taught in U.S. Patents Nos.: 1,172,270 to Franzie; 2,040,943 to Kang; 2,289,416 to Fine; 3,255,015 to Blanchon; 3,395,019 to Kviesitis; 3,930,027 to Kelly et al.; 3,950,543, to Buffa et al. and 4,311,714 to Goering et al.

However, in the processes of these patents, the enzymatically treated ground grains or grain fractions are not taught as being formed into flaked, shredded, or extruded cereals.

The production of instant breakfast cereals in powdered or flaked form involving the enzymatic treatment of ground grains or grain fractions with sugar producing enzymes is disclosed in U.S. Patent Nos. 4,374,860 to Gasser et al and 4,438,150 to Gantwerker.

The enzymatic treatment of ground grains or grain fractions with sugar-producing enzymes in the production of a ready-to-eat (RTE) cereal in shredded, flaked, or extruded form is disclosed in U.S. Patents Nos.: 1,564,131 to Kellogg; 1,568,162 to Humphrey; and 4,431,674 and 4,435,430 to Fulger et al.

In the production of these ready-to-eat cereals and instant breakfast products using sugar producing enzymes, enzymatic treatment so as to retain starch or starch and high molecular weight dextrins for matrix forming ability while developing sweetness and taste complexity is not taught.

The enzymatic conversion of whole cereal grains with sugar producing enzymes in the preparation of cereal products is disclosed in: U.S. Patent No. 2,310,028 to Gustavson; U.S. Patent No. 2,555,235 to Huzenlaud; U.S. Patent No. 2,627,464 to Keahetian; U.S. Patent No. 3,948,015 to Gay; U.S. Patent No. 4,371,551 to Fulger et al and Japanese Patent Publication No. 37-1654.

However, in these processes for producing cereal products by enzymatically converting whole cereal grains with sugar producing enzymes, forming enzymatically treated grains into ready-to-eat cereals in shredded, flaked, or extruded form is not taught.

Production of a ready-to-eat cereal in shredded or flaked form involving enzymatic treatment of whole cereal grains with sugar producing enzymes is disclosed in U.S. Patents Nos.: 2,174,982 to Kellogg; 2,289,416 to Fine; and 4,254,150 to Fritze et al.

U.S. Patent No. 2,174,982 teaches a process for making shredded or flaked cereal foods from cereal grains such as wheat, rye, corn or oats. Here, the whole grain is boiled in alkali to partially dissolve the bran coating. The grain is then washed to remove the alkali and then treated with malt to convert starch to dextrins. A flavoring substance is added to the wort, whereupon the wort is kept at 148°F-170°F to expand the grain and allow penetration of the flavoring substance. The material is then cooked under pressure, dried and either flaked or shredded. The moisture content of the material for shredding is 20 percent. The dextrins in the product, it is taught, make it crisper.

U.S. Patent No. 2,289,416 teaches a process for making cereal foods in flaked or shredded form from whole grains. Prior to enzymatic action, the whole grains are treated by rupturing of the bran coat and gelatinization of starch with heat. Following this pretreatment, enzymes are added directly to the whole grain. The enzymes, it is taught, act more rapidly on gelatinized starch. Malted grain is taught as an enzyme source. Efficient conversion of gelatinized starch is taught as being at a temperature of from 60°C to 70°C. Bumping, it is taught, greatly increases the permeability of the endosperm which can be performed before or after the gelatinization step. The addition of enzyme to flour and to bumped grain prior to cooking is taught as resulting in no substantial conversion of starch to subcomponents. Malted barley flour and a diastase concentrate, i.e., an Aspergilius oryzae amylase preparation, are each used as enzyme sources. Maltose is the only specifically disclosed sugar which is produced by this process.

U.S. Patent No. 4,254,150 teaches the production of cereal foods in flaked form. Here, starch, in situ, is converted to dextrose by enzymatic conversion. Whole grains or grains ground from coarse to medium-fine grist is mixed with water to form a mash with the grain fraction of the mash being 20 to 40 percent. The mash is allowed to swell whereupon alpha-amylase is added. The pH of the mash is adjusted. The mash is then heated in a steam injection cooker at a temperature in the range of 100°C to 110°C. The mash is then passed through a tube type converter which is also maintained at a temperature of 100°C to 110°C. At this point the starch is converted to maltodextrin. The enzyme amyloglucosidase is then added to the mash to effect the conversion of maltodextrin to dextrose. The mash is then dried on a single roll dryer to form a thermoplastic film. The film is cooled to make it fryable and then comminuted to form flakes. Prior to drying the mash, bran or other additives may be added for the production of a fodder. The enzymatic degradation of the starch in the grain is achieved without separating the starch from the grain or from the other dry substances such as gluten, fibers, and husks.

In the production of these ready-to-eat cereals, the enzymatic treatment of the whole cereal grains with sugar reducing enzymes is not taught as retaining starch and high molecular weight dextrins for their matrix forming properties while developing sweetness and taste complexity.

The present invention provides a process for the production of breakfast cereals which are enzymatically sweetened while retaining starch and high molecular weight dextrins for their matrix forming properties. In the present invention, the in situ production of fructose requires less starch conversion to achieve a desired level of sweetness. The production of fructose also provides enhanced, honey-like, graham, flavors and aromas in the breakfast cereals of the present invention. Reduced starch breakdown provides for improved formability of the enzymatically treated cereal grains into breakfast cereal shapes and improved shape retention at a given level of sweetness. The starch and/or cellulose component of a cereal grain, a flour or meal may be enzymatically altered to produce fructose in the present invention. Thus, in one aspect of the invention, the cellulose component of a flour or meal comprising starch and cellulose fiber, is enzymatically altered to saccharify the cellulose and to produce fructose in the treated flour or meal under conditions in which substantially all of the fructose so produced and all, or a substantial portion, of the starch are retained.

## SUMMARY OF THE INVENTION

The present invention provides a process for the production of breakfast cereals which are sweetened by the enzymatic conversion of starch and/or cellulose to fructose. Conversion to fructose provides enhanced, honey-like, graham flavors and aromas to the cereal products of the present invention. In addition, to achieve a desired level of sweetness, less conversion of starch or less breakdown of high molecular weight dextrins is needed because of the higher sweetening power of fructose compared to other reducing sugars. Accordingly, a larger proportion of starch and higher molecular weight dextrins can be retained for matrix formation or machinability of the enzymatically treated cereal grains. In addition, shape retention of the breakfast cereals is enhanced at a given sweetness level as the proportion of retained starches and/or high molecular weight dextrins increases.

In the present invention, cereal grains or at least one cereal grain fraction is cooked with water to at least partially gelatinize the cereal starch. The enzymatic treatment may begin prior to cooking, simultaneously with cooking, or subsequent to cooking. The amount of water used during the enzymatic treatment is preferably limited so that at least substantially all of the water is absorbed by the cereal grain thereby reducing loss of sugars upon draining of the cooked grains. The cooking of the cereal grains or the cereal grain fraction and the enzymatic treatment are performed so that the discreteness or integrity of the grains or particles is substantially retained. This permits formation of the enzymatically treated particles into breakfast cereal shapes using conventional, mass production cereal forming equipment.

In one aspect of the present invention, glucoamylase is used to form dextrose. A portion of the dextrose is converted to fructose by the use of glucose isomerase. The glucoamylase and glucose isomerase are added simultaneously or sequentially. The glucoamylase and glucose isomerase are preferably added after cooking to avoid premature inactivation of these enzymes. In the present invention, alpha-amylase may be used to convert the starch to dextrins. The alpha-amylase may be added simultaneously with or prior to addition of the glucoamylase and glucose isomerase.

In another aspect, the invention relates to a process for producing a fructose-sweetened cereal product comprising the steps of:
providing a cereal comprising cereal fiber, moistening the cereal comprising cereal fiber, contacting said moistened cereal comprising cereal fiber with a series of enzymes comprising cellulase and glucose isomerase, incubating said moistened cereal comprising cereal fiber and enzymes for a sufficient period of time to produce fructose and collecting a sweetened cereal product comprising fructose. A cereal flour or meal is preferred as the cereal comprises cereal fiber.

In another aspect of the invention, the process is carried out contacting the moistened cereal flour or meal with cellulase, saccharifying enzymes such as α-amylase and amyloglucosidase and glucose isomerase.

In another aspect, the invention relates to a process for producing a sweetened cereal product in which the collected sweetened cereal product is baked.

In yet another aspect, the invention relates to a process for producing a sweetened cereal product in which the cereal flour or meal is moistened with a sucrose solution prior to incubation with the series of enzymes to produce fructose.

In still another aspect, the invention relates to a process for producing a sweetened cereal comprising hydrolysing a portion of or substantially all of the cellulose in said cereal with cellulase and converting the products of said hydrolysis to fructose with glucose isomerase.

The enzymatically treated cereal grains may be incubated or tempered up to about 48 hours, typically from about 2 to about 24 hours. The tempered product is then drained, and formed into breakfast cereal shapes. The enzymatically treated cereal grains may be formed by shredding, flaking, grinding, extrusion, and the like. The enzymes are inactivated by heating of the formed cereal. Enzyme inactivation may also be initiated prior to or simultaneously with the forming step.

The amount of fructose produced by the enzymatic conversion of the starch is sufficient to provide a sweet taste in combination with the other reducing sugars produced during the enzymatic treatment. The fructose which is produced also provides a honey-type or graham cracker-type taste and aroma. The fructose content of the cereal products of the present invention is at least about 1% by weight, preferably at least about 5% by weight, based upon total dry solids. The reducing sugar content of the cereal products of the present invention may range up to about 35 percent by weight, suitably from about 10% to about 25% by weight, based upon total dry solids. The amount of fructose which is produced by the enzymatic conversion may range from about 5% to about 45% by weight, suitably from about 15% to about 40% by weight, based upon the total weight of monosaccharides of the cereal product.


## DETAILED DESCRIPTION OF THE INVENTION


The cereal products of the present invention are naturally sweetened with reducing sugars comprising fructose. Naturally occurring starches and/or cellulose components present in cereal grains or cereal grain fractions are enzymatically altered to provide sweetening and taste complexity and pleasant aromas in the final product.

The cereal grains which may be used in the present invention are wheat, oats, rice, corn, barley, rye, combinations thereof, and the like. The preferred cereal grain is wheat. The grains may be bumped or unbumped. Bumped grains provide for more rapid penetration by the enzymes which results in shorter conversion times. Cereal grain fractions which may be used in the present invention include any comminuted products or meals derived from cereal grains. They include a bran fraction, an endosperm fraction, a germ fraction, portions thereof, or mixtures thereof such as flour or whole wheat flour. The cereal grain fraction may be obtained by conventional milling, classification, and blending processes.

The at least one cereal grain fraction should generally contain at least 10% by weight of cereal starch, suitably from about 25% to about 45% by weight, on a dry weight basis. The preferred cereal grain fraction is wheat bran. The starch content of the bran may range up to about 60% by weight of naturally occurring and/or added cereal starch. A higher starch content, suitably from about 45% to about 55%, permits the production of higher amounts of fructose in the cereal product.

The cereal grains may be cut, suitably steel cut, whole cereal grains, or germinated or malted grains. Whole berries are preferred for the production of shredded cereals and flaked cereals.

In the present invention, the enzymatic conversion is conducted so as to retain the discreteness or integrity of the cereal grains. It is also preferred to maximize the amount of fructose as a percentage of the total reducing sugars provided it does not create excessive sweetness, or adversely affect machinability or product color. In the treatment of a cereal grain fraction, the integrity of the particles, such as bran, should also be retained. Retention of the integrity or discreteness of the cereal grains or particles and retention of starch or high molecular weight dextrins is needed for formability or machinability into breakfast cereal shapes on conventional processing equipment. For example, in the production of shredded wheat, the enzymatic treatment of whole berries should not destroy the integrity or discreteness of the berry. If discreteness is destroyed, the grains tend to clog shredding roll feed hoppers and tend to stick to the shredding rolls. The retention of starch or matrix-forming high molecular weight dextrins, should be

6

sufficient so as to provide machinability and formability as well as to provide resistance to breakage in the final cereal product.

In the present invention, D-xylose ketol-isomerase, also known as xylose isomerase, but more commonly known as glucose isomerase is used to convert glucose to fructose. In one aspect of the present invention, the glucose is produced by the use of amyloglucosidase or glucoamylase, also known as amylo-1,6-glucosidase. The glucoamylase may produce glucose by enzymatic conversion of starch or by enzymatic conversions of dextrins derived from the cereal grain starch. The dextrins may be obtained by enzymatic treatment of the cereal grains or at least one cereal grain fraction with an alpha-amylase. Alpha-amylases hydrolyze starch molecules at alpha-1,4-hemiacetal (-C-O-C-) links randomly, whereas cleavage by glucoamylase yields glucose. Accordingly, cleavage by glucoamylase produces more reducing sugar while retaining higher molecular weight dextrins for their matrix forming ability. In the present invention, the use of alpha-amylase is optional.

In the present invention, the optional alpha-amylase is admixed with the cereal grains or at least one cereal grain fraction and water either prior to cooking, at the initiation of cooking, or after cooking. When the alpha-amylase is added prior to cooking, the cereal grains or at least one grain fraction is soaked in the presence of alpha-amylase suitably at a temperature of from about 68°F (20°C) to about 212°F (100°C). Suitable soaking times range from about one-half hour to about four hours.

The cereal grains are cooked in the presence of water and the optional alpha-amylase to at least partially gelatinize the starch. The degree of gelatinization is typically complete. By complete gelatinization it is meant that there is a complete absence of birefringence and complete absence of enthalpy of gelatination by differential scanning calorimetry.

Cooking temperatures generally range from about 17°F (80°C) to about 212°F (100°C). Cooking times generally range from about 15 minutes to about 45 minutes. The pH during presoaking or cooking is suitably from about 5 to about 8. Generally the cooking times and temperatures should be sufficient to completely eliminate white centers or to leave only faint white centers in the berry.

After cooking, the cereal grains or at least one cereal grain fraction and the water are admixed with glucoamylase and glucose isomerase. The optional alpha amylase may also be added after cooking. When it is added after cooking, this may be done in addition to previously added alpha amylase. The glucoamylase and glucose isomerase may be added after cooking either simultaneously or sequentially, or both. Sequential addition of these enzymes provides for tailoring of pH and temperature to the particular enzyme. When alpha amylase is added after the cooking step, it may be prior to or simultaneously with the addition of the glucoamylase.

When the glucoamylase and glucose isomerase are added sequentially, the enzymatic treatment with glucoamylase is suitably conducted at a temperature of from about 68°F (20°C) to about 176°F (80°C) and a pH of from about 3 to about 8, preferably from about 131°F (55°C) to about 167°F (75°C) and a pH of from about 4 to about 6. The enzymatic treatment with the glucose isomerase is then suitably conducted at a temperature of from about 68°F (20°C) to about 212°F (100°C) and at a pH of from about 5 to about 9, preferably from about 131°F (55°C) to about 158°F (70°C), and a pH of from about 6 to about 8.

When the enzymatic treatment with glucoamylase and glucose isomerase is simultaneous, the conversion is suitably conducted at a temperature of from about 68°F (20°C) to about 176°F (80°C) and a pH of from about 5 to about 8, preferably at a temperature of from about 131°F (55°C) to about 158°F (70°C), and a pH of about 6 to 7. When alpha amylase is added after cooking, the treatment with the alpha amylase may suitably be up to about 3 hours, suitably at about 176°F (80°C) to about 194°F (90°C) prior to addition of the glucoamylase. After treatment with alpha-amylase, the reaction mixture is permitted to cool, as needed, to a temperature suitable for use with glucoamylase. The total treatment time or incubation period with glucoamylase and glucose isomerase is suitably up to about 48 hours, typically from about 2 hours to about 24 hours. When glucoamylase and glucose isomerase are used sequentially, treatment times may range up to about 24 hours with the glucoamylase and up to an additional 24 hours with the glucose isomerase.

In the process of the present invention, tempering of the cereal grains or at one least one cereal grain fraction occurs after cooking and during the enzymatic treatment with the optional alpha-amylase, the glucoamylase and the glucose isomerase.

The amount of water used during cooking and during enzymatic treatment with the glucoamylase and the glucose isomerase is preferably limited so that at least substantially all of the water is absorbed by the cereal grains or at least one cereal grain fraction. This reduces loss of reducing sugars to drain water. Suitably, the amount of water used during cooking and enzymatic treatment ranges from about 20% by weight to about 55% by weight based upon the total weight of water and cereal grains or cereal grain fractions. Water may be added after cooking for admixture of enzymes with the cooked cereal grains or grain fraction so as to achieve homogeneity during enzymatic treatment. After enzymatic treatment, any additional water remaining is drained. The drained, enzymatically treated cereal grains or grain fraction may optionally be tempered to distribute water substantially uniformly throughout the cereal grains prior to forming.

Unless indicated to the contrary, enzyme concentrations for use in the present invention are defined in terms of activity as in U.S. Patent No. 4,376,824 at column 8 line 47 to column 11 line 8. The determination of saccharides, calculation of dextrose equivalent (DE), and determination of the degree of isomerization (% fructose) are also in accordance with the methods described in U.S. Patent 4,376,824 unless otherwise indicated. See column 11 line 10 to column 12 line 43, herein incorporated by reference.

Thus, for purposes of the present invention, alpha-amylase concentration is expressed as liq/g where "g" is the grams of dry substance starch. "Liq" is short for liquefons which is an enzyme activity defined by a modification of Standard Test Method, AATCC 103-1965 "Bacterial alpha-amylase Enzymes Used in Desizing, Assay of" published in the 1967 Edition of Technical Manual of the American Association of Textile Chemists and Colorists, Volume 43, pp. B-174 and B-175.

The modifications of the published method are:

(1) The buffer solution for the starch substrate is prepared by dissolving 25.3g of c.p. sodium hydroxide and 340 g of c.p. potassium dihydrogen phosphate in water and diluting to 2-liters.

(2) 125 ml of the buffer solution is added to the cooled, pasted starch substrate before the substrate is brought to the 500 ml volume.

(3) The pH of the starch substrate is determined and, if necessary, adjusted to $6.20 + 0.05$.

(4) A 0.025 molar calcium chloride solution is used for enzyme sample dilution. This is prepared by dissolving 11.1 g of anhydrous c.p. calcium chloride in water and bringing the volume to 4 liters.

(5) The formula for converting from BAU TO liquefons is BAU x 2.85 = liquefons.

A gluccamylase activity unit (GU) is defined as the amount of enzyme which catalyzes the production of one gram of dextrose per hour at $60°$ C at pH 4.5 in the procedure described below.

10 ml of a 10 percent solution of a partially hydrolyzed starch (such as Maltrin-10, a product of Grain Processing Co., Muscatine, Iowa), containing 20 mM acetate buffer at pH 4.5, is pipetted into a capped reactor maintained at $60°$ C. One ml of a glucoamylase solution, containing 0.03 to 0.15 GU is added and mixed therein, and the mixture maintained for one hour at $60°$ C. At the end of the one-hour incubation period, enzyme action is stopped by adding a predetermined volume of 1 M sodium hydroxide so as to obtain a pH of 8.5 to 10.5. The mixture is then cooled to room temperature.

2.5 ml of the assay hydrolysate so obtained is pipetted into 25 ml of Fehling's solution prepared as described in method E-26 for DE determination in "Standard Analytical Methods of the Member Companies of the Corn Industry Research Foundation, Inc.", 1001 Connecticut Ave., N.W., Washington D.C. 20036. (Dextrose equivalent or DE is defined as the concentration of reducing sugars present expressed as dextrose and calculated as a percentage of the dry substance.) The mixture is brought to a boil and titrated with standard dextrose solution containing 5 g of dextrose per liter according to the procedure cited above for DE determination. A control mixture is prepared and titrated in the exact same manner as for the assay hydrolysate above except that the 1 ml of glucoamylase solution is added to the substrate solution after the one-hour incubation period and after the addition of sodium hydroxide solution. Glucoamylase activity is calculated as:

$$\frac{GU}{g} = 0.002 \ V((C-A)/W)$$

where V is the total volume (ml) of assay hydrolysate (usually 11.2 ml); C is the ml of standard dextrose solution used in the titration of the control mixture; A is the ml of standard dextrose solution used in the titration of the assay hydrolysate; and W is the weight of enzyme per ml of the diluted enzyme solution.

Glucose isomerase activity is expressed as IGIU units.

IGIU is the abbreviation for International Glucose Isomerase Unit and is that amount of enzyme which will convert 1 micromole of glucose to fructose per minute in a solution initially containing 2 moles of glucose per liter, 0.02 moles of $MgSO_4$ and 0.001 mole of $CoCl_2$ per liter at a pH of 6.84 to 6.85 (0.2 M sodium maleate) and at a temperature of $60°$ C. Glucose isomerase determinations were carried out by the method described by N. E. Lloyd, et al., Cereal Chem., 49, No. 5, pp. 544-553 (1972).

Unless indicated to the contrary, enzyme concentrations or dosages are expressed as liq/g, GU/g, or IGIU/g where "g" is the grams of dry substance starch initially present. Unless indicated to the contrary, it is assumed that wheat has a starch content of 63.2% by weight on a dry basis.

In the present invention, suitable enzyme concentrations are from about 1 liq/g to about 1000 liq/g for alpha-amylase, from about 0.1 GU/g to about 10 GU/g for glucoamylase, and from about 1 IGIU/g to about 100 IGIU/g for glucose isomerase. If alpha amylase is used, its concentration is preferably at least about 200 liq/g.

Enzymes for use in the present invention are commercially available. Heat stable enzymes are preferred. Suitable enzymes for use in the present invention include heat-stable alpha-amylase which is available from Novo Industry A/S, glucoamylase sold under the registered trade mark of SPEZYME GA-200, which is available from Finnish Sugar Co., Ltd., and glucose isomerase also available from Finnish Sugar Co., Ltd. and sold under the trademark SPEZYME GI.

The pH during the enzymatic treatment may be controlled with an edible buffer. An acetate buffer comprising a mixture of acetic acid and acetate is preferred. The pH may also be adjusted continuously by the use of a pH adjuster such as sodium hydroxide, potassium hydroxide, or calcium carbonate. Other buffers or pH adjusters which may be used include propionates, lactates, fumarates, malates, citrates, and phosphates, such as potassium phosphate.

The present invention also concerns a process for making a sweetened cereal product in which a portion of the cellulose fraction, or both the starch and cellulose components of cereal flour or meal are converted into fructose. Prior to milling, cereals have high contents of fiber and starch. Such cereals include a variety of large and small grains including corn, rye, rice, wheat and barley. Other food crops, including soy beans and peanuts, have significant starch and fiber content that may be converted into fructose by the general method according to the invention. Cereals are particularly high in fiber or cellulose as well as containing a significant amount of starch. For example, the pericap, nucellus and tegmen of wheat, all of which together comprise bran, are high in fiber and comprise a significant portion of graham and whole wheat flour. Bran is separated from the endo-sperm of wheat kernels in the milling process to form bread flour and the bran may be collected and separately milled to produce a bran flour. The fiber components of these flours contain significant amounts of cellulose.

In the process according to the invention a moistened milled cereal comprising cereal fiber, or whole cereal grain, preferably a cereal flour or meal comprising cellulose and starch is optionally disrupted and contacted with a series of enzymes which comprises cellulase and glucose isomerase and optionally will further include saccharifying enzymes; the moistened cereal flour or meal with the series of enzymes is incubated for a sufficient period of time to produce fructose, and the cereal product containing fructose is collected.

In the process according to the invention, a substantial portion of the cellulose content of the cereal flour or meal may be enzymatically converted to fructose. The conversion of the cellulose portion of the cereal flour or meal into glucose, and the production of fructose therefrom, leaves intact a significant amount of the starch content of the cereal flour or meal which can be used to maintain texture and formability of the cereal product ultimately produced from the treated flour or meal. Alternatively, a portion of the starch may also be saccharified, in addition to producing glucose or other sugars or with the enzymatic action of glucose isomerase, additional fructose, while still maintaining a significant amount of starch and high molecular weight dextrins which may be desirable for texturing and forming operations of the treated cereal.

Fructose is known to have a higher degree of sweetness than glucose or sucrose. Thus, high fiber cereals may be produced having a desired degree of sweetness while retaining a relatively low digestible calorie content since it is not necessary to saccharify starch in one aspect of the process according to the invention. Accordingly, one product of the process of the invention is an enzymatically sweetened-low-calorie-high fiber cereal product.

In greater detail, the process according to several aspects of the invention entails the production in a moistened cereal of fructose by enzymatic treatment with cellulase and glucose isomerase. The cereal will generally be milled or subjected to some process whereby the cereal grain is fractured, crushed, or disrupted into small pieces. Such milled cereal grain is preferably in the form of a coarse or fine flour or meal produced from a starch and cellulose containing cereal grain. Such grains include large and small grains including corn, rice, wheat, oats, barley and rye. The various flours and meals that may be produced from these grains are the material used for the process according to the invention. Flours and meals produced from these grains and which are appropriate for the production of breakfast cereals are generally known. With respect to flours, those produced from wheat are preferred, and in particular, bran flours, graham flour and whole wheat flour are appropriate. Bran flour is especially preferred.

In general, the flour will be supplied in dry form having a water or moisture content of 20% or less, in general about 10%. Prior to or concurrent with enzymatic treatment according to the invention, the moisture content of the flour is increased. In general, the moisture content is increased sufficiently to permit the activity of the series of enzymes mentioned above, all of which in general function in an aqueous or hydrated environment. Moisture content is determined as the weight percent $H_2O$ of the total mixture. The moisture content will range from about 25% and up. A moisture content of between about 40% and about 80% is preferred for sustaining the activity of the series of enzymes mentioned above.

The moisture content may be varied depending upon the type of flour used, the texture of the final cereal product desired, and the speed of the saccharification and fructose formation reactions desired. However, in general, the moisture content will not exceed the amount of water that the flour or meal can absorb. At a moisture content between about 55 and 60% using a bran flour, a friable sweetened cereal product is obtained.

The moisture content of the flour or meal is adjusted by the addition to or removal of water from the flour. If the flour or meal is presoaked in an aqueous solution or in water prior to the addition to the enzyme series, it may be desirable to remove water by ccnventional means such as decanting, blotting, pressing, straining, and heating or a combination of these ccnventional means. If a dry flour or meal is used, moisture content may be adjusted by the addition of solutions containing water. Such a solution may include aqueous enzymes, including the cellulases and glucose isomerase mentioned above, or optionally, in addition, the amylases and amyloglucosidases.

Alternatively, or in addition, the flour may be moistened by aqueous flavoring syrup. Such flavoring syrups may contain sucrose, fruit extracts, and the like. The addition of such aqueous sucrose solutions, however, is not required in the process according to the invention, as substantial amounts of glucose and fructose are developed from the flour by enzymatic action.

It is preferred in the process according to the invention to conduct the enzymatic treatment of the cereal flour or meal with a moisture content sufficient to sustain the activity of the series of enzymes. However, in general, it is not desirable to add so much water to the cereal grain or flour that excess water may be recovered from the moistened grain or flour by draining after the enzymatic treatment. Moisture contents so great as to permit drainage of excess water will generally lead to a loss of soluble sugars produced as a result of the enzymatic treatment. Therefore, it is desirable to moisten the cereal grain or flour with no more solution of water than the cereal grain or flour can completely absorb.

In preparing flour or meal for further processing, the flour or meal is disrupted to partially alter the starch and cellulose contained in the flour or meal. In general, the disruption process involves the application of heat, and preferably moist heat. Thus, disruption may be carried out by steaming, steam injection, pressure cooking in a closed vessel or, less preferably, by the application of dry heat. Chemical disruption processes such as treatment with mild alkali are also known.

The addition of the enzyme series to the whole cereal grain, cereal meal or flour can occur at various points in the process for producing the sweetened cereal product according to the invention. In general, the enzyme may be added before, after, or during disruption of the flour or meal. It is generally preferred to contact the cereal grains, flour or meal with the series of enzymes after disruption. Without being bound by this theory, the disruption of the cereal grains, flour or meal is believed to increase the microaccessability of enzyme to substrate in the disrupted flour and thus maximizes both the fructose yield and the availability of sugars and smaller saccharides for browning reactions if the sweetened cereal product is eventually cooked.

The series of enzymes may alternately be added prior to disruption. If the series of enzymes is added at this point in the process, the subsequent disruption step can be adjusted to maintain enzyme activity for a portion of the disruption cycle by adjusting the rate at which the temperature of the enzyme-cereal grain, flour or meal mixture is raised. Of course, the disruption step may be used to eventually substantially or completely inactivate the series of enzymes added to the whole cereal grain, flour or meal if the series of enzymes is added prior to the disruption step.

The series of enzymes may alternately be added during the disruption step, the amount of each enzyme added and the rate of heating may be adjusted to obtain the desired level of fructose formation in the final product. In each case, when the series of enzymes is added before or during the heat disruption step, and the heat of the disruption step is used to increase the activity of the series of enzymes, it may be desirable to use thermostable enzymes. Such thermostable enzymes are generally known and may be obtained from microorganisms that are found in high thermal environments. A thermostable glucose isomerase and glucoamylase has been described, for example, in U.S. Patent Nos. 4,532,208 and 4,536,477, respectively. Furthermore, the use of thermostable enzymes permits the process of the invention to be carried out at temperatures generally higher than 75°C and thus may be useful in shortening the time necessary fcr producing fructose in the sweetened cereal product.

The order in which the members of the series of enzymes is added to the moistened cereal flour or meal is also significant in the process according to the invention. In general, the enzymes may be added to the disrupted or non-disrupted grains, flour or meal all at one time or in an order wherein the glucose isomerase enzyme is added last. It is desirable to achieve a steady state of glucose isomerase activity similar to that achieved by a slow feed of glucose for conversion into fructose. The concentration of fructose produced under these conditions is not so great as to inhibit the formation of fructose from glucose. In general, when the flour substrate is in a moisture condition of 75% water or less, it is desirable to add all the enzymes at once. Under these conditions, glucose produced by cellulase or by the combination of cellulase and saccharifying enzymes such as α-amylase and amyloglucosidase is converted to fructose by glucose isomerase at a rate essentially equivalent to the rate at which glucose is produced. In general, it is desirable to add an excess of glucose isomerase.

After enzymatic treatment, the cereal grains or at least one cereal grain fraction may then be formed into breakfast cereal shapes by using conventional mass production cereal processing equipment. For example, the sweetened cereal grains may be shredded, flaked, extruded, or ground.

In the production of a ready-to-eat shredded cereal biscuit, suitable moisture contents of the enzymatically sweetened cereal grains for shradding range from about 28% to about 49%, more typically from about 39% to about 43% by weight, based upon the weight of the cereal grain. The cooked and tempered enzymatically sweetened cereal grains are transferred, suitably by means of belt conveyors to a hopper which feeds a screw conveyor. The latter transfers the cereal grain to a series of shredding rolls or mills via flow tubes or hoppers.

The shredding mills comprise a pair of rolls that rotate in opposite directions. One of the rolls has circumferential grooves and crosshatching grooves which are transverse to the circumferential grooves for the production of an integral net-like sheet. The spacing between the rolls is preferably controlled so as to avoid the production of webbing. Upon passing between the rolls, the cereal grain is deformed into the circumferential grooves and the crosshatching grooves. Each pair of rolls produces a cereal dough layer having a plurality of generally parallel longitudinal strands and a plurality of crosshatchings generally perpendicular to the strands. The longitudinal strands are produced by the circumferential grooves and run in parallel with the direction of movement of an underlying conveyor. The crosshatchings of the dough layer are produced by the crosshatching grooves and run generally perpendicular to the direction of movement of the conveyor.

The shredding mills are arranged in a linear series along the common underlaying conveyor. Each of the shredded dough layers or sheets are deposited on the conveyor in super-position, with their longitudinal strands running in the same direction.

The shredded cereal dough layers are continuously laminated. The laminate is cut transversely and longitudinally to the direction of flow of the product into multiple lines of biscuit preforms using known cutting devices. The cutting can be completely through the laminate to form the individual biscuit shapes prior to baking. However, cutting partially through the filled laminate to form biscuit shapes, followed by baking and separating the baked partially cut laminate into individual biscuits in known manner is preferred. This procedure provides easier control of the orientation of a cut product as it passes through the oven.

In the production of a flaked cereal, the enzymatically sweetened cereal grains may be dried to a suitable flaking moisture content and passed between large steel counter rotating cylinders having smooth surfaces. The cylinders may be internally cooled or heated. The sweetened cereal grains may be subjected to grinding prior to flaking.

Ground cereal products may be produced by subjecting cereal grains or a cereal grain fraction to drying followed by grinding in a mill. The milled product may then be toasted.

In the production of extruded products, the enzymatically sweetened cereal grains or at least one cereal grain fraction may be optionally dried to a suitable extrusion moisture content and extruded using a twin screw cooker-extruder. Various dies may be used to extrude the sweetened material into breakfast cereal shaped pieces which may be puffed or unpuffed.

In the present invention, the enzymes are inactivated to provide a shelf stable product suitably by heating during conventional baking, toasting, and drying steps. For example, in the production of a shredded biscuit product, the cut laminate may be dried, baked and toasted in conventional equipment. Suitable ovens for drying, baking and toasting the laminate include Proctor and Schwartz, Werner-Lehara, and Spooner ovens containing forced air and gas fired burners and a conveyor. Temperature profiles used in the oven for drying, baking and toasting of the biscuit preforms are generally within the range of about 200°F to about 600°F. Temperatures within this range are generally suitable for total enzyme inactivation. The total time for drying, baking and toasting should be such so as to avoid excessive browning, particularly in view of the presence of the reducing sugars in the products of the present invention. Suitable times for drying, baking and toasting will depend upon the product thickness, product size, oven type, and amount of reducing sugars in the product. Suitable times generally range from about 4 minutes to about 10 minutes.

The amount of fructose produced in the enzymatically saccharified cereal products of the present invention should be sufficient to provide sweetness and a pleasing, complex honey-like flavor. The amount of fructose produced by the enzymatic treatment of the cereal grains or at least one cereal grain fraction should be at least 1% by weight, preferably at least about 5% by weight, based upon the total dry weight of the cereal product. It is believed that the pleasing, complex flavors and aromas of the products of the present invention are the result of Maillard reactions between fructose and cereal grain proteins. The cereal products of the present invention may have a reducing sugar content up to about 35% by weight, suitably from about 10% by weight to about 25% by weight, on a dry basis. The fructose content of the cereal products of the present invention typically ranges from about 5% to about 45%, preferably from about 15% to about 40% by weight or more, based upon the total monosaccharide content of the cereal product.

The reducing sugar content of enzymatically treated wheat samples may be determined by using a modification of the dinitrosalicylic acid (DNS) procedure of Bernfeld. Other methods for measuring reducing sugars could also be used.

To prepare samples for analyses they are ground to a relatively fine powder with a small electric coffee grinder. After grinding and blending a subsample is taken for dry solids determination.

For extraction of soluble reducing sugar a 0.3-1.0 g d.b. sample of the ground wheat is suspended in 50 ml of deionized water and stirred for two hours. Insoluble material is then removed by high speed centrifugation or filtration. Aliquots of the clear supernate or filtrate are used directly for the DNS reducing sugar procedure.

For moist samples enzyme inactivation is needed before extraction. This can be accomplished by adjusting the pH of the extraction to about 10 by adding a few drops of 10% NaOH immediately after adding the water to the ground wheat sample, or by extracting the ground sample in 50 ml of 0.02 M carbonate-bicarbonate buffer, pH 10.

The DNS reagent is prepared by dissolving 1.0 g 3.5-dinitrosalicylic acid (DNS) in 20 ml 2 N NaOH and 50 ml deionized water and adding 30 g Rochelle salt (potassium sodium tartrate). When all of the salts have dissolved water is added to a final volume of 100 ml. The reagent is stored in a stoppered brown bottle at room temperature.

A standard glucose solution (0.01 M) is prepared by dissolving 0.180 g anhydrous glucose in about 80 ml deionized water and then diluting to 100 ml. From the standard glucose solution a series of dilutions ranging in concentration from 0 to 10 uM/ml is prepared. A series of test tubes each containing 1.0 ml DNS reagent is then prepared. Into each tube is pipetted 1.0 ml of a glucose solution. Each tube is capped with a glass ball (marble) or aluminum foil and immersed in a boiling water bath for exactly five minutes. The tubes are then placed in a cold water bath for 10 minutes. Then, 10.0 ml deionized water is added to each tube and mixed well with a vortex mixer. Absorbance at 540 nm is read in a suitable colorimeter or spectrophotometer against a reagent blank (1.0 ml DNS, 1.0 ml deionized water, treated as above). The absorbance at 540 nm vs. glucose concentrations is then plotted.

Unknown samples are diluted to 1-10 uM reducing sugar/ml. Each unknown sample is treated as in the standardization procedure. Reducing sugar concentration is determined from the standard curve.

The amount of cereal starch which is converted to dextrins and reducing sugars may range up to essentially 100% by weight on a starch dry basis. However, the conversion to low molecular weight products should not be so high so as to destroy formability or machinability or to reduce final cereal product strength. In addition, the conversion to low molecular weight products should be sufficiently high so as to provide a sweetening amount of fructose.

The present invention may be used for the production of ready-to-eat breakfast cereals, instant cereals, and hot cereals.

The present invention is further illustrated in the following examples. Unless otherwise stated, enzyme dosages or concentrations are per gram of dry substance wheat starch, assuming a starch content for wheat of 63.2% by weight on a dry basis. Also, all temperatures are in degrees C, and all percentages, ratios, and proportions are by weight and the wheat is unbumped unless indicated to the contrary:

## EXAMPLE I

Ready-to-eat shredded cereal biscuits naturally sweetened with fructose may be made by cooking 1000 grams of unbumped, whole wheat berries with 700 ml of water at 100° C for 30 minutes, followed by cooling the cooked wheat to 85° C without draining. Novo Termamyl T-120 alpha-amylase (20,000 liq/ml) may then be added to the cooked wheat in an amount of 3.2 ml (about 100 liq per gram of bone dry starch). The alpha-amylase and cooked wheat berries are then mixed thoroughly and kept at 85° C for two hours, and then cooled to 60°C for simultaneous addition of glucoamylase and glucose isomerase. Spezyme glucoamylase (200 GU/ml) may be added in an amount of 1.6 ml (about 0.5 GU per gram of bone dry starch). Spezyme glucose isomerase (630 IGIU/ml) may be added in an amount of 1.0 ml (about 1.0 IGIU per gram of bone dry starch). The wheat berries and the enzymes are mixed and then held at 60°C for 18 hours. The enzymatically treated cereal grains may then be permitted to drain. The cereal grains may then be dried to a moisture content of about 41%. The cereal grains may then be shredded using counter-rotating shredding rolls, one of which is grooved, to form continuous net-like sheets. The net-like sheets may then be laminated to form a laminate of 10 layers. The laminate may then be partially cut into rectangular, spoon-sized biscuit preforms. The preforms may then be dried, baked, and toasted in a gas-fired zone oven for about 7 minutes at temperatures ranging from inlet to outlet within the range of about 200°F to about 600°F, and then separated into individual biscuits to obtain a product having a moisture content of about 4.5% by weight.

EXAMPLE II

Ready-to eat shredded cereal biscuits naturally sweetened with fructose may be made as in Example I except after addition of the glucoamylase and glucose isomerase, the wheat berries and enzymes may be held at 60°C for two hours instead of 18 hours.

EXAMPLE III

Ready-to-eat shredded cereal biscuits naturally sweetened with fructose may be made as in Example I except the 3.2 ml (100 liq/g) of alpha-amylase may be added to the cook water, followed by addition of the wheat berries. After the cook, the wheat berries are held at 85°C for two hours, and cooled to 60°C for addition of glucoamylase and glucose isomerase as in Example I.

EXAMPLE IV

Ready-to-eat shredded cereal biscuits naturally sweetened with fructose may be made as in Example III except after addition of the glucoamylase and glucose isomerase, the wheat berries and enzymes may be held at 60°C for two hours instead of 18 hours.

EXAMPLE V

Ready-to-eat shredded cereal biscuits naturally sweetened with fructose may be made as in Example I except: a) the cooked wheat may be cooled to 21°C instead of 85°C without draining, b) the alpha-amylase is omitted, and c) the cooled wheat is heated to 60°C after about 2 hours at 21°C for addition of the glucoamylase and glucose isomerase.

EXAMPLE VI

Ready-to-eat shredded cereal biscuits naturally sweetened with fructose may be made as in Example II except: a) one half of the alpha-amylase (50 liq./g) is added to the cook water, followed by addition of wheat berries, and the remaining half of the alpha-amylase (50 liq./g) is added to the cooked wheat, b) the glucoamylase is added in an amount of 3.2 ml (about 1.0 GU/g) instead of 1.6 ml (about 0.5 GU/g), and c) the glucose isomerase is added in an amount of 0.5 ml (about 0.5 IGIU/g) instead of 1.0 ml (about 1.0 IGIU/g).

## EXAMPLE VII

Ready-to-eat shredded cereal biscuits naturally sweetened with fructose may be made as in Example II except: a) the wheat berries may be presoaked with 1.6 ml of the alpha-amylase (50 liq./g), and b) the amount of the alpha-amylase added to the cooked wheat is reduced from 3.2 ml (100 liq./g) to 1.6 ml (50 liq./g).

## EXAMPLE VIII

Ready-to-eat shredded cereal biscuits naturally sweetened with fructose may be made by cooking 1000 grams of unbumped, whole wheat berries with 700 ml of water at 210° F for 30 minutes, followed by cooling the cooked wheat to 60° C without draining for addition of glucoamylase and glucose isomerase. Spezyme glucoamylase (200 GU/ml) may be added in an amount of 32.0 ml (about 10.0 GU per gram of bone dry starch). The glucoamylase and wheat berries are mixed and then held at 60° C for about 4 hours. Then, Spezyme glucose isomerase (630 IGIU/ml) in an amount of 5.0 ml (about 5.0 IGIU per gram of bone dry starch) is buffered to a pH of 7.0 with a 1.0M potassium phosphate buffer. The mixture of cooked wheat berries and glucoamylase is admixed with the buffered glucose isomerase then held at 60° C for 14 hours. The enzymatically treated cereal grains may then be permitted to drain. The cereal grains may then be dried to a moisture content of about 41%, and shredded, cut, dried, baked, and toasted as in Example I.

## EXAMPLE IX

Ready-to-eat cereal flakes naturally sweetened with fructose may be made by drying the drained, enzymatically sweetened cereal grains of Example VIII to a moisture content of about 20% by weight instead of 41%, flaking the dried wheat berries between flaking rolls, and then toasting the flakes at a temperature of about 275° F in a toasting drum oven to inactivate the enzymes and to produce a toasted, fructose sweetened product.

## EXAMPLE X

This Example demonstrates the potential loss of reducing sugars which are produced during enzymatic cooking of wheat in an amount of water which is substantially greater than can be absorbed by the wheat.

Twenty grams of clean, soft, white wheat, i.e., about 18.46 g d.b., was mixed with 27 mls of water. The water was dosed with 0.185 ml of Termamyl T-120 alpha amylase containing 1850 liquefons. This mixture of wheat, water and enzyme was immersed in a boiling water bath for 30 minutes. A control was also set up which contained no alpha-amylase but was otherwise treated in the same manner as described above. After cooking, the wheat was drained for 15 minutes in a sintered glass funnel and aspirated for an additional 30 minutes under laboratory vacuum. The liquid drained from the wheat was collected and analyzed for reducing sugar. Next, the wheat samples were ground in a laboratory mill and thoroughly blended. The material was then sampled for a dry solids determination. Next, the blended material was extracted exhaustively with water and the extracts were analyzed for reducing sugar by the dinitrosalicylic acid procedure of Bernfield. [Bernfield P. (1955) in Method in Enzymology (Colowick, S.P. and Kaplan, H. O., ed.) Vol. 1, p. 149, Academic Dress, New York] The Bernfield procedure was calibrated against anhydrous dextrose. The data collected from this procedure are set out in Table 1:

TABLE 1

|  | Reducing Sugar(% db.) | | |
| --- | --- | --- | --- |
|  | Ground Wheat | Drain Water | Total |
| Control | 0.51 | 0.02 | 0.53 |
| alpha-amylase Treated | 0.54 | 0.17 | 0.71 |

Enzymatic hydrolysis of starches results in producing soluble reducing sugars which tend to leach into the aqueous solution surrounding the wheat. This loss of reducing sugar is undesirable in terms of potential sweetening effect and, additionally, causes an increase in the biological oxygen demand of the process drain water.

EXAMPLE XI

This example demonstrates the effect of various alpha-amylase treatments on whole wheat starch hydrolysis.

Six trials were performed wherein the water added for the cooking in each assay was reduced from 1.35 mls per gram of wheat (Example X) to 0.76 mls. per gram of wheat. Additionally, three different strategies for alpha-amylase addition and hydrolysis were employed, each using two different alpha-amylase dosages (Termamyl T-120), i.e., 100 and 200 liq/g starch. In trials 1 and 2, whole, clean wheat was added to preheated water followed by the addition of alpha-amylase. The mixture was immersed in a boiling water bath and cooked for 30 minutes. After cooking, the wheat was drained into a sintered glass funnel for 15 minutes and aspirated with laboratory vacuum for an additional 30 minutes. A dry solids determination was made upon the sample. The wheat was then suspended in a 20 millimolar acetate buffer, having a pH of 3.0. The material was then ground in a blender to prepare samples which were assayed for reducing sugar concentrations. The drain water was also analyzed for reducing sugar.

In trials 3 and 4, the wheat was cooked in the same manner as in trials 1 and 2. After the cooking step, the undrained wheat was allowed to cool to 80°C and incubated for two hours at 80°C before draining, drying, and blending. In trials 3 and 4, no drain water was collected because the wheat retained all the moisture.

In trials 5 and 6, the wheat was presoaked at room temperature after the addition of alpha-amylase. After three hours of soaking, the wheat was cooked and handled as in trials 1 and 2. In trials 1, 3 and 5 the alpha-amylase concentration was 100 liq/g and in trials 2, 4 and 6 the alpha-amylase concentration was 200 liq/g. The results of trials 1 through 6, showing approximate enzyme treatment times, are illustrated in Table 2.

TABLE 2

| EFFECT OF VARIOUS ALPHA-AMYLASE TREATMENTS ON WHEAT STARCH HYDROLYSIS | | | | | | |
| --- | --- | --- | --- | --- | --- | --- |
| Trial | Dosage Liq/g | Drain Vol. ml | Reducing Sugar % db. | | | Time (hrs) |
|  |  |  | Wheat | Drain | Total |  |
| 1 | 100 | 2.3 | 0.32 | 0.05 | 0.37 | 1.25 |
| 2 | 200 | 2.5 | 1.34 | 0.06 | 1.40 | 1.25 |
| 3 | 100 | 0 | 0.83 | 0 | 0.83 | 2.5 |
| 4 | 200 | 0 | 1.88 | 0 | 1.88 | 2.5 |
| 5 | 100 | 2.0 | 0.83 | 0.10 | 0.93 | 4.25 |
| 6 | 200 | 3.2 | 2.05 | 0.14 | 2.19 | 4.25 |

Trials 5 and 6, involving presoaking at room temperature for 3 hours, produced the most total reducing sugar. It is believed the presoaking allows the wheat to imbibe moisture and alpha-amylase before being subjected to the cooking temperatures. It is noted, however, that a significant portion of the reducing sugar was lost in the drain water in this procedure and enzyme contact times were the longest. In contrast, trials 3 and 4, where the cooked wheat was incubated at 80°C before the drain and dry step resulted in slightly lower degrees of hydrolyses with no loss of either drain water or reducing sugar with substantially shorter contact times. Thus, it has been shown that thermo-stable alpha-amylase can be used effectively in the wheat cooking step to hydrolyze part of the wheat starch to dextrins.

EXAMPLE XII

This example demonstrates the effect of various glucoamylase treatments on hydrolysis of alpha-amylase treated whole wheat starch.

Five trials were performed including a control. In each trial, 20 grams of wheat was dosed with alpha-amylase (Termamyl T-120) at a concentration of 200 liq/g starch and soaked for one hour at room temperature before a 30-minute cook in a boiling water bath using about 0.75 ml of water per gram of wheat. In the control trial, the wheat was drained and dried after the cook. In the rest of the trials, the undrained wheat was allowed to cool to 58°C, whereupon the cooled undrained wheat was dosed with 1.0 ml glucoamylase (Spezyme GA) containing 58.3 GU or a dosage of 5 GU/g dry wheat starch. This mixture was then incubated for various periods of time at 58°C before draining and drying. After sampling for dry solids determinations the wheat was ground, suspended in 20 millimolar acetate buffer having a pH of 3, and boiled to terminate enzyme activity. The material was then centrifuged to remove insoluble material. The various supernatants were analyzed for reducing sugar. The results of Example III are set out in Table 3:

TABLE 3

| Trial | Reducing Sugar % db | Percent Hydrolysis* |
|---|---|---|
| Control - No GA | 0.77 | 1.1 |
| GA - 2hrs. | 12.36 | 17.6 |
| GA - 4 hrs. | 13.21 | 18.8 |
| GA - 10 hrs. | 14.61 | 20.8 |
| GA - 22 hrs. | 16.91 | 24.1 |

* Based on a starch content of 63.2% (702 mg potential reducing sugar per gram of wheat.)

The results depicted in Table 3 show that the glucoamylase was rapidly absorbed by the cooked wheat. After only two hours of incubation at 58°C a reducing sugar level of 12.36% db. was obtained. Thus, about 17 percent of the starch was hydrolyzed to reducing sugar. Longer incubations resulted in further reducing sugar generation. At 22 hours, the starch was about 24 percent hydrolyzed.

Portions of the wheat treated with glucoamylase for four or ten hours were ground and dried in a 90°C forced-air oven for one hour. The drying samples emitted a pleasant sweet aroma somewhat reminiscent of honey. A sample of wheat which had been cooked in the conventional manner without addition of enzymes was also dried in a similar manner. The dried, treated samples had a rich golden-brown color, while the untreated sample retained a pale straw color after drying. The taste of the treated sample was perceptibly sweeter and more complex than that of the untreated sample or a commercial shredded wheat sample.

EXAMPLE XIII

This example demonstrates the production of sweetening amounts of reducing sugars using lower alpha-amylase and glucoamylase concentrations than in Example XII by the additional use of glucose isomerase.

Six trials were run where the cooking step was performed with reduced water levels of about 0.75 ml of water per gram of wheat.

In trial 1, no alpha-amylase was added for the cooking step. After cooking, the wheat was cooled to 60°C, dosed with 1.0 ml of enzyme solution containing 58.3 GU of glucoamylase (Spezyme GA) (0.5 GU/g) and 11.6 IGIU of glucose isomerase, (Spezyme GI) (1 IGIU/g), and incubated for two hours at 60°C.

In trial 2, no alpha-amylase was added for the cooking step. The wheat was cooked and then incubated for two hours at 60°C with glucoamylase at a concentration of 2.0 GU/g and glucose isomerase at a concentration of 2.5 IGIU/g.

In trial 3, alpha-amylase was added to the cooking step in a concentration of 100 liq/g. The wheat was cooked and then incubated for two hours at 60°C with glucoamylase at a concentration of 0.5 GU/g and glucose isomerase at a concentration of 1.0 IGIU/g.

In trial 4, alpha-amylase (Termamyl T-120) was added to the cooking step at a concentration of 100 liq/g. The wheat was cooked and then incubated for two hours at 60°C with glucoamylase at a concentration of 2.0 GU/g and glucose isomerase at a concentration of 2.5 IGIU/g.

In trial 5, both alpha-amylase and glucoamylase were added to the cooking step at a concentration of 100 liq/g and 0.5 GU/g respectively. The wheat was cooked and then incubated for two hours at 60°C without further enzyme addition.

In trial 6, alpha-amylase was added to the cooking step in the concentration of 200 liq/g. The wheat was cooked and then incubated for two hours at 60°C with glucoamylase at a concentration of 5 GU/g and glucose isomerase at a concentration of 5 IGIU/g.

In each trial, after the two-hour incubation at 60°C, the wheat was divided into two equal portions. The first portion was immediately drained and dried at 90°C in a forced-air oven. The second portion was tempered at room temperature for 16 hours before draining and drying. Portions of each of the dried samples were ground and sampled for solids determinations and reducing sugar concentrations. The results are depicted in Table 4:

## TABLE 4

| Trial | Enzyme Conc. | | | Percent of Reducing Sugar* | |
|-------|--------------|------|------------|-----------------|-----------|
|       | Alpha (liq/g) | GA (GU/g) | GI (IGIU/g) | Non-Tempered | Tempered |
| 1 | 0 | 0.5 | 1.0 | 2.0 | 2.8 |
| 2 | 0 | 2.0 | 2.5 | 3.0 | 5.0 |
| 3 | 100 | 0.5 | 1.0 | 2.6 | 4.8 |
| 4 | 100 | 2.0 | 2.5 | 3.9 | 6.7 |
| 5 | 100 | 0.5 | 0 | 2.2 | 3.9 |
| 6 | 200 | 5.0 | 5.0 | --- | 13.2 |

* Grams of reducing sugar (expressed as glucose) per 100 grams dried wheat.

The results show that the sugar content of the tempered samples was consistently higher than that of the non-tempered samples. This result indicates that some enzyme activity remained after the 100°C cook and 60°C incubation steps. Samples of the ground, dried wheat were tasted and compared to a sample of ground commercial shredded wheat. The perceived sweetness of each sample correlated reasonably well with the reducing sugar content of each sample. The sugar content for the 18-hour sample from trial 6 is equivalent to about 1/2 teaspoon of sugar per 1 ounce serving of cereal. The sweetness of the sample was readily apparent and may be greater than that needed for a naturally sweetened cereal. The 18-hour sample from trial 5, where no glucose isomerase was used and the glucoamylase was added to the cook, had virtually no perceptible sweetness. The use of glucose isomerase results in perceptible sweetness at lower reducing sugar levels than when glucoamylase is used without it as in trial 5 and in Example XII.

## EXAMPLE XIV

This example demonstrates the increase in reducing sugar production over that obtained in Example XIII when: a) alpha-amylase treatment after cooking is conducted at 85°C instead of 60°C and, b) glucoamylase and glucose isomerase treatment is at 60°C for 18 hrs instead of 2 hrs followed by 16 hours at room temperature.

In trial 1, a control was run where no enzyme was added. The wheat was cooked with about 1.39 ml of water per gram of wheat at about 100°C for 30 minutes.

In trial 2, no enzyme was added for the cooking step. The wheat was cooked and then incubated for two hours at 85°C with alpha-amylase (Termamyl T-120) at a concentration of 100 liq/g. After the incubation step, the mixture was allowed to cool to 60°C whereupon 1.0 ml of solution containing 58.3 GU of Spezyme GA glucoamylase (0.5 GU/g) and 11.7 IGIU of Spezyme GI glucose isomerase (1.0 IGIU/g) was added to the cooled mixture. The cooled mixture was then incubated at 60°C for either 2 or 18 hours.

In trial 3, alpha-amylase was added to the cooking step at a concentration of 50 liq/g. After the cooking step, more alpha-amylase was added to the mixture at a concentration of 50 liq/g which was then allowed to incubate for two hours at 85°C. The mixture was cooled to 60°C and the glucoamylase and glucose isomerase were added as in trial 2.

In trial 4, wheat was presoaked with alpha-amylase at a concentration of 50 liq/g for two hours at 60°C. After the cooking step, more amylase was added at a concentration of 50 liq/g and the wheat was treated as in trial 3.

In trial 5, alpha-amylase was added to the cooking step at a concentration of 100 liq/g. After the cooking step, the wheat was incubated for two hours at 85°C without further alpha-amylase addition. The wheat was then allowed to cool to 60°C and glucoamylase and glucose isomerase were added as in trial 2.

In trial 6, alpha-amylase was added to the cooking step in the concentration of 50 liq/g. After the cooking step, additional amylase was added to the mixture at a concentration of 50 liq/g which was then incubated for two hours at 85°C. The mixture was then allowed to cool to 60°C where upon glucoamylase at a concentration of 1.0 GU/g and glucose isomerase at a concentration of 0.5 IGIU/g were added to the mixture which was then allowed to incubate at 60°C for either 2 or 18 hours.

In trials 2 through 5 the amount of water used for cooking was about one-half of the water level used in trial 1. In trial 6, the amount of water used for cooking was about two-thirds of the water level used in trial 1.

After treatment as set out in trials 1 through 6 the wheat was drained and dried for two hours in a forced-air oven at 90°C. The wheat was then ground and blended whereupon samples were taken for dry solids determinations, reducing sugar analyses and sensory evaluations. The results are depicted in Table 5.

TABLE 5

| Trial | Dosage | | | Percent of Reducing Sugar | |
|---|---|---|---|---|---|
| | (liq/g) | GA(GU/g) | GI(IGIU/g) | 2 Hours | 18 Hours |
| 1 | 0 | 0 | 0 | 0 | --- |
| 2 | 100 | 0.5 | 1.0 | 11.1 | 17.2 |
| 3 | 100 | 0.5 | 1.0 | 8.7 | 12.7 |
| 4 | 100 | 0.5 | 1.0 | 9.7 | 14.2 |
| 5 | 100 | 0.5 | 1.0 | 6.9 | 14.3 |
| 6 | 100 | 1.0 | 0.5 | 14.0 | 21.5 |

The results of Example XIV show that significant hydrolysis of the starch in the wheat to reducing sugars was achieved in all of the assays except the control. This was especially true after 18 hours of incubation. The most reducing sugar was produced in trial 6 where more than 21 percent reducing sugar was produced. All of the 18 hour samples except trial 1, had readily apparent sweetness and pleasing, complex, honey-like flavors. It is believed that the flavors are the result of Maillard reactions between reducing sugars, especially fructose, and other components in the cooked grain during the high temperature drying.

## EXAMPLE XV

This example demonstrates the effect of glucoamylase dosage on reducing sugar production in whole wheat with or without alpha-amylase addition to the cook.

In this investigation eight trials were set up. Trials 1, 2 and 3 were set up in the absence of any alpha-amylase. All the trials of this Example were low water cooks, i.e., 75 ml of water to 100 gms of wheat. After the cooking step, the wheat was cooled to 60° C and dosed with 1.0 ml of Spezyme GA glucoamylase solution containing the appropriate amount of enzyme activity. In the first trial, glucoamylase was added at a concentration of 2 GU/g . In trial 2, gluco amylase was added at a concentration of 5 GU/g. In trial 3, glucoamylase was added at a concentration of 10 GU/g. The wheat was allowed to incubate at 60° C with the glucoamylase and periodic samples were taken for reducing sugar analyses.

In trials 4 and 5, alpha-amylase (Termamyl T-120) was added for the cooking steps at a concentration of 50 liq/g. In trial 4, glucoamylase was added at a concentration of 2 GU/g. In trial 5, glucoamylase was added at a concentration of 5 GU/g. As with trials 1, 2 and 3, the wheat was incubated at 60 C and periodic samples were taken for reducing sugar analyses.

In trials 6 and 7, alpha-amylase was added to the cooking step at a concentration of 100 liq/g. After the cooking step in trial 6, the wheat was cooled to 60° C and dosed with glucoamylase at a concentration of 2 GU/g. In trial 7, the wheat was cooled to 85° C and held for three hours before cooling to 60° C, whereupon glucoamylase was added at a concentration of 2 GU/g and periodic samples were taken for reducing sugar analyses.

In trial 8, alpha-amylase was added to the cooking step at a concentration of 200 liq./g. After the cooking step, the wheat was cooled to 60° C and dosed with glucoamylase at a concentration of 2 GU/g. The wheat was incubated at 60° C and periodic samples were taken for reducing sugar analyses. Table 6 presents the reducing sugar percentages for trials 1-8:

TABLE 6

| Assay | Dosage | Percent Reducing Sugar | | | | |
|-------|--------|--------|--------|--------|--------|--------|
|       |        | 3 Hrs. | 6 Hrs. | 10 Hrs. | 22 Hrs. | 46 Hrs. |
| 1 | 0 liq - 2 GU | 2.88 | 6.54 | 8.72 | 16.61 | 18.09 |
| 2 | 0 liq - 5 GU | 3.51 | 6.76 | 12.23 | 19.87 | 26.67 |
| 3 | 0 liq - 10 GU | 4.70 | 10.28 | 15.73 | 23.95 | 36.39 |
| 4 | 50 liq - 2 GU | 3.12 | 5.45 | 7.98 | 16.33 | 17.41 |
| 5 | 50 liq - 5 GU | 3.34 | 7.35 | 11.18 | 16.70 | 31.00 |
| 6 | 100 liq - 2 GU | 3.14 | 6.51 | 8.37 | 13.81 | 24.84 |
| 7 | 100 liq - 2 GU | 7.07 | 13.64 (7 Hrs.) | - | 21.29* | 35.21** |
| 8 | 200 liq - 2 GU | 4.11 | 6.82 | 10.74 | 21.74 | 30.11 |

\* 19 hours
\*\* 43 hours

These data indicate that significant amounts of reducing sugar can be produced without the use of alpha-amylase. Low doses of alpha-amylase, i.e., 50 liq/g. or less, had no significant effect on the amount of reducing sugar produced by a constant glucoamylase dosage. In trials 1-3, where no alpha-amylase was used and significant levels of reducing sugars were produced, a glucoamylase dosage of only 2 GU/g of starch, produced 16.6 percent reducing sugar, i.e., 16.6 gms. per 100 gms. of wheat, in 22 hours at 60° C. Higher glucoamylase dosages increased the rate of production of reducing sugars.

The results of trials 3-8 show the effect of various alpha-amylase dosages upon reducing sugar production when used in combination with glucoamylase. With lower alpha-amylase dosages, i.e., 50-100 liq/g. reducing sugar production tended to be about the same or decrease when alpha-amylase was used with a 60° C incubation. For example, reducing sugar production with a glucoamylase dosage of 2 GU/g was about the same with or without the alpha-amylase addition (trials 1,4,and 6). Reducing sugar production was increased with a higher alpha-amylase dosage of 200 liq/g. or with a dosage of 100 liq/g. if a 3-hour 85° C incubation step was included after the cooking step and prior to the addition of any glucoamylase.

In trial 7, an amylase dosage of 100 liq/g. and an 85° C incubation step produced more than 21 percent reducing sugar in 19 hours incubation with a glucoamylase concentration of 2 GU/g. The 19-hour sample from trial 7 was found to have a very sweet taste. An incubation at 85° C as in trial 7, followed by a shorter, 60° C incubation of only 7-10 hours may be used to produce a sufficiently sweet grain.

### EXAMPLE XVI

This example demonstrates the effect of glucose isomerase and pH on reducing sugar distribution when alpha-amylase is not added for the enzyme saccharification of wheat.

In this example, two enzyme-treatment trials were performed without alpha-amylase addition. Twenty grams of whole wheat was cooked at about 100°C in an amount of water of about 0.75 ml per gram of wheat for 30 minutes.

In trial 1, after the cooking step, the wheat was cooled and incubated 16 hours at 60°C solely with glucoamylase at a dosage of 10 GU/g of starch by addition of 1 ml glucoamylase solution containing 200 GU per ml.

In trial 2, after the cooking step, the wheat was incubated at 60°C with both glucoamylase and glucose isomerase at doses of 10 GU/g and 10 IGIU/g, respectively. Two milliliters of glucose isomerase (100 IGIU/ml) was added in a 1.0M phosphate buffer, at pH of 7.0.

In trial 2 the glucoamylase was added first, whereupon the mixture was incubated for 16 hours at a pH of about 5.3 to 5.8 before adding the glucose isomerase. This strategy was used to allow the glucoamylase enzyme to hydrolyze some of the starch to glucose under more optimal pH conditions before adding the isomerase at the higher pH. After incubation with glucose isomerase for 24 hours at 60°C, the wheat was oven-dried, ground and then sampled for analyses of sugars by liquid chromatography. The results are presented in Table 7:

### TABLE 7

| Trial | Carbohydrate Composition % of Wheat dry basis | |
|---|---|---|
| | Dextrose | Fructose |
| 1 | 16.97 | 0.78 |
| 2 | 10.91 | 6.13 |

### EXAMPLE XVII

This example demonstrates the preparation of an enzyme sweetened bran cereal, formed by extrusion.

100 grams of Red Wheat bran (about 14% by weight starch, dry basis) and 100 grams of White Wheat heavy bran (about 60% by weight starch, dry basis) were mixed together. The starch content was about 37% by weight based upon the total weight of the brans (dry basis). After adding 200 grams of water, the mass was cooked at 100°C for 30 minutes to gelatinize the starch. After cooling to 65°C, Spezyme GA200 glucoamylase (200 GU/gram) was added in an amount of 5 grams (about 5 GU/gram bran). The glucoamylase and bran were mixed and then held for 60 minutes at 65°C. Then, sufficient magnesium hydroxide (about 1 g/100 g bran) was added to adjust and maintain the pH at about 7.5 after which 0.6 ml Spezyme GI glucose isomerase (3600 IGIU/ml) was added to give an enzyme concentration of about 10.8 IGIU/gram bran. The mixture of bran and enzyme was then held, for an additional 60 minutes at 65°C. The sweetened bran cereal was then cooked at 121°C for 15 minutes and extruded through 1/8″ holes using a Hobart A200 mixer equipped with a meat grinder/extruder. The strands so formed were dried at 100°C to produce the final product with a moisture content of about 1.5% to about 3% by weight. The dried product had a sugar content, by liquid chromatography, of about 11% glucose, 5% fructose, 3% maltose, and 1% other sugars, with sufficient residual starch and high molecular weight dextrin (about 17%) for formability. The residual starch and high molecular weight dextrin content was calculated by difference, based upon the 37% by weight starch content of the bran mixture (dry basis).

### EXAMPLE XVIII

White wheat bran was soaked in distilled deionized water for approximately 60 hours at 5°C, was squeeze dried through cheese cloth, and ground to a fine mush. 0.25 grams of the wetted bran was placed in a 50 ml closed vial and enzyme was added as shown below. Total volume was 10 ml and the final pH was 5 or 6 adjusted with 1 NHCl. The reaction was run in duplicate at 40°C with shaking for 3.0 hours. At the end of the reaction, the sample was analyzed for carbohydrate content using a Boehringer-Mannheim food analysis kit (catalogue No. 139106). The enzyme preparations were:

A cellulase enzyme preparation (referred to as CEL in some of the following tables) from Trichoderma reseii in 50 mM sodium acetate, 3 mM sodium azide buffer at pH 5.5 with a total specific activity of approximately 104 units/ml was used. Shoemaker, S. et al, "Characterization and Properties of Cellulases Purified From Trichoderma reseii Strain L27," Bio/Technology (Oct. 1983). This enzyme preparation contains the following types and amounts of enzymes: cellobiohydrolase I (CBHI), 60%; CBHII, 10%; endoglucanse I (EGI) 3.6%; EGII, 3.0%; beta-glucosidase I (BGI), 0.05%; and BGII, 2.0%. Enzyme activity was determined by incubating the enzyme preparation with the following substrates: 1% Avicel, phosphoric acid swollen cellulose (PSC), xylan (Sigma) and mannan in 50 mM sodium acetate buffer, pH 4.5 at 45°C for 30 minutes. Soluble reducing sugar was measured as sugar equivalents. Units of activity were expressed as umole glucose (or xylose) equivalents per minute. Activity on carboxymethylcellulose (CMC, Hercules) was determined by incubating enzymes with 0.27% CMC in 50 mM acetate buffer, pH 4.5 for 30 minutes and measuring the decrease in viscosity versus time with a rotating viscometer (Brookfield). Units of CMC activity were arbitrarily expressed as change in fluidity per minute. Cellobiose and paranitrophenyl-D-glucoside (PNPG) activities were determined according to Emert, Doctoral Thesis, Va. Polytech. Inst. and State U., Blacksburg, Va. (1973), with units of activity expressed as umole cellobiose consumed per minute and umole PNP released per minute, respectively. The cellulase enzyme had the following total activity distribution (U/mg): Avicel (crystallized cellulose) 0.3; phosphoric acid swollen cellulose (PSC) 3.6; carboxymethyl cellulose 3.6; cellobiose 0.3; paranitrophenyl-D-glucoside (PNPG) 0.3; Xylan 1.2; Mannan 0.3.

Glucose isomerase (Spezyme) preparation in 50 mM sodium phosphate, 10 mM magnesium sulfate, 1mM $CoCl_2$, pH 6.0 having an activity of 110 IGIU/ml was used. Results are shown in Table 8.

TABLE 8

| Reaction | Bran(g) | CEL | GI | Buffer | Avg. Yield (gm/L) | | % Dry Weight Fructose |
|---|---|---|---|---|---|---|---|
| | | | | | D-Glucose | D-Fructose | |
| 1 | 0.25 | -- | -- | 10 ml | 0 | 0 | |
| 2 | 0.25 | 5 ml | -- | 5 ml | .534 | 0 | |
| 3 | 0.25 | 5 ml | 5 ml | -- | .50 | 0.052 | ~9% |

An additional 5 ml aliquot of glucose isomerase was added to Reaction vessel 3. Reaction vessels 2 and 3 were incubated at 05°C overnight without shaking. In addition, a duplicate set of flasks identical to Reaction 3 was run overnight at 05°C without shaking. Yields were as follows:

| Reaction | D-Fructose | D-Glucose | % Dry Weight Fructose |
|---|---|---|---|
| 2 | 0 | .534 | 0 |
| 3 (+GI) | .12 | .47 | ~20% |
| 3 (duplicate) | 0.05 | .71 | ~6.6% |

EXAMPLE XIX

The following reactions were run using the same enzyme preparation as in Example XVIII at 45°C without shaking overnight one flask per preparation. Both white and red wheat bran flour, prepared as in Example XVIII were used:

| 1. White bran flour | | | | | | | |
|---|---|---|---|---|---|---|---|
| Reaction | Bran Flour | Cel | GI | Buffer | Glucose | Fructose | % Dry Weight Fructose |
| 1 | 0.25 gm | -- | -- | 10 ml | 0 | 0 | |
| 2 | -- | 5 ml | 5 ml | -- | 0 | 0 | |
| 3 | 0.25 gm | 5 ml | -- | 5 ml | .86 | 0 | |
| 4 | 0.25 gm | 5 ml | 5 ml | -- | .86 | 0.99 | ~9.3% |

| 2. Red Bran Flour | | | | | | | |
|---|---|---|---|---|---|---|---|
| Reaction | Bran Flour | Cel | GI | Buffer | Glucose | Fructose | % Dry Weight Fructose |
| 1 | 0.25 | -- | -- | 10 | 0 | 0 | |
| 2 | -- | 5 ml | -- | 5 | 0 | 0 | |
| 3 | 0.25 | 5 ml | -- | 5 | .66 | 0 | |
| 4 | 0.25 | 5 ml | 5 | -- | .62 | .08 | ~11.4% |

In both experiments the reaction products containing glucose or glucose/fructose were darker than the reaction products without glucose or fructose.


## EXAMPLE XX


The enzymes used in the following reaction were indicated in Example XVIII. Reactions were run at a total volume of 20 ml in a 250 ml Erlenmeyer flask. The bran flour was not prewashed. Temperature was 45° C for 20 hours then 60° C for four hours; no shaking for 20 hours, then 75 rpm for four hours.

| Reaction | Bran (g) | ML | | | Yield g(L) 24 hr. | | |
|---|---|---|---|---|---|---|---|
| | | Cellulase | GI | Buffer | Glucose | Fructose | |
| 1 | 5 | 5.5 | 1.1 | 13.4 | 11.0 | 10.8 | |
| 2 | 5 | -- | -- | 15 | 0 | 0 | |
| 3 | -- | 5.5 | 1.1 | 13.4 | 0 | 0 | |
| Time course of yield for Reaction (g/l) | | | | | | | |
| | Hours | | | | | | |
| | 0 | 1 | 2 | 3 | 20 | 22 | 24 |
| D-glucose | 0.9 | 4.0 | 5.1 | 4.6 | 9.9 | 10.3 | 11.0 |
| D-fructose | 0.06 | 2.2 | 3.5 | 4.3 | 9.4 | 10.1 | 10.8 |

Assuming an approximate cellulose content of 11.3% the percent conversion of cellulose to glucose and fructose is 77%.


## EXAMPLE XXI


Enzymes, buffers and bran flour were the same as in Example XIX. In addition, the following enzymes were used: α-amylase (1,4-α-D-glucan-glucanohydrolase E.C. 3.2.1.1) from B. licheniformis (Sigma) (referred to herein as αA), amyloglucosidase, exo 1,4 α-glucosidase 1,4 α-D-Glucan glucohydrolase E.C. 3.2.1.3 from Asoergillus oryzae (Sigma) referred to here as GA). α-Amylase concentration was 11040 U/ml, GA was 1400 U/ml, GI was 2250 IGIU/ml, and cellulase was 18.9-227 U/ml. Buffer was the same as that used for glucose isomerase: 50 mM NaPO₄, pH 6.0, 10 mM MgSO₄, 1 mM CoCl₂. A flavoring syrup having the following composition was used in parts per hundred (ppC): liquid sucrose (67 brix) 34 ppC; NaCl 2.0; fig and prune concentrate 2 ppC; vitamin solution including niacinamide, B1, B6, B2 and C .8 ppC; balance water.

The following protocol was used: Into a 1 liter beaker, bran (102.5 gm) and flavor syrups (46.6 gm (41 ml) were measured. The material was thoroughly mixed and loosely covered with aluminum foil. The mixture was autoclaved at 121°C, 15 psi for 25 minutes and then ccoled to room temperature. The mixture was distributed in 39 gm aliquots into 1 liter beakers. The aliquots were treated as follows:

A

Cellulases    2500 U (7 ml)
Glucose isomerase    2500 U (1.6 ml)
Buffer, pH 5.5    11.4 ml

Incubated at 45°C, no shaking, moisture saturated atmosphere, tightly covered with foil.

B

α-amylase    2500 U (0.23 ml)
Amyloglucosidase    2500 U (1.8 ml)
Glucose isomerase    2500 U (1.6 ml)
Cellulases    2500 U (7 ml)
Buffer, pH 5.5    9.37 ml

C

Buffer, pH 6.0    20 ml

Incubated at 60°C in a water bath, no shaking, tightly covered with foil.

Samples were taken periodically and frozen. Assay for D-glucose and D-fructose were carried out on 0.2 gm of each samples as described above. Dry weights of various samples were determined by heating to 150°C for two hours in aluminum pans using a drying oven.

D-glucose/D-fructose analyses: These were done as described in Examples XVIII through XX. The bran used for the study had a moisture content 50-60%. Therefore, each reaction beaker contained 23.1 gm dry bran; and each sample analyzed was 0.1084 gm, the bulk of which was dry bran.

Results: Production of D-glucose/D-fructose:

1. Reaction A (cellulases/glucose isomerase)

|  | gm/L | | | | |
| --- | --- | --- | --- | --- | --- |
|  | 1 hr. | 2.hr. | 5 hr. | 22 hr. | 24 hr. |
| D-glucose | 12.4 | 12.0 | 8.3 | 12.2 | 15.0 |
| D-fructose | 1.2 | 1.6 | 2.2 | 4.2 | 5.1 |
| % conversion of cellulose 0.084 gm aliquot | ~72 | ~72 | ~53 | ~86 | ~106 |

2. Reaction B (amylases/cellulases/glucose isomerase)

| | gm/L | | | | |
|---|---|---|---|---|---|
| | 1 hr. | 2.hr. | 5 hr. | 22 hr. | 24 hr. |
| D-glucose | 19.4 | 19.9 | 13.3 | 18.8 | 21.6 |
| D-fructose | 2.4 | 4.1 | 4.5 | 4.9 | 9.6 |
| % conversion of cellulose 0.084 gm aliquot | ~34 | ~37 | ~28 | ~43 | ~48 |

3. Reaction C (control) No D-glucose or D-fructose.

The mixtures in the enzyme-treated instances were darker and stickier than the control, due to the high monosaccharide content and browning reactions. They had a pleasant aroma.

Samples of beakers of Reactions B and C contents were baked for 20 minutes at 150°C. The resultant products were very different in obvious properties. The aromas of the enzyme-treated samples were much more intense and pleasant than that of the control. Also, the enzyme-treated samples were darker and particles adhered to each other, whereas the control was lighter and particles were discrete.

## EXAMPLES XXII through XXV

Fructose sweetened cereal products may be produced as in Examples XVIII through XXI except the sodium azide and $CoCl_2$ are eliminated.

## Claims

1. A method for producing an enzyme-saccharified cereal product, comprising:

a) cooking cereal grains or at least one cereal grain fraction with water to at least partially gelatinize cereal starch;

b) treating the cooked cereal grains or at least one cereal grain fraction with water and enzymes comprising glucose isomerase to produce fructose;

c) forming the enzyme treated cereal grain or the at least one enzyme treated cereal grain fraction; and

d) inactivating the enzymes, the amount of fructose produced being sufficient to provide a sweet taste to the cereal product.

2. A method as claimed in Claim 1 wherein whole cereal grains are cooked in step (a), and the cooked grains retain discreteness during the enzymatic treatment of step (b).

3. A method as claimed in Claim 2 wherein the discrete enzymatically treated cereal grains are formed by shredding.

4. A method as claimed in Claim 2 wherein the discrete enzymatically treated cereal grains are formed by flaking.

5. A method as claimed in Claim 2 wherein the discrete enzymatically treated cereal grains are formed by grinding.

6. A method as claimed in Claim 1 wherein the at least one enzymatically treated cereal grain fraction or the enzymatically treated cereal grains are formed by extrusion.

7. A method as claimed in Claim 1 wherein the amount of water present during the enzymatic treatment of the cereal grains or at least one grain fraction is limited so that at least substantially all of the water is absorbed by the cereal grain or the at least one grain fraction to retain fructose in the cereal product.

8. A method as claimed in Claim 7 wherein the amount of water present during the enzymatic treatment ranges from about 20 percent by weight to about 55 percent by weight based upon the total weight of the water and the weight of the cereal grains or at least one grain fraction.

9. A method as claimed in Claim 1 wherein the amount of fructose produced by the enzymatic treatment is at least about 5% by weight, based upon the total dry solids of the cereal product.

10. A method as claimed in Claim 1 wherein the enzymes comprise glucoamylase and glucose isomerase.

11. A method as claimed in Claim 1 wherein the enzymatic treatment and the cooking are partially simultaneous.

12. A method as claimed in Claim 11 wherein the enzymatic treatment comprises cooking the cereal grain or at least one grain fraction in the presence of alpha-amylase to convert a portion of the cereal starch to dextrins, and then subjecting the cooked product to glucoamylase and glucose isomerase to produce fructose.

13. A method as claimed in Claim 12 wherein prior to cooking, the cereal grain or at least one grain fraction is soaked in the presence of alpha-amylase.

14. A method as claimed in Claim 12 wherein the cooked product is subjected to glucoamylase and glucose isomerase simultaneously.

15. A method as claimed in Claim 12 wherein the alpha-amylase is used in an amount of from about 1 liq/g to about 1,000 liq/g., glucoamylase is used in an amount of from about 0.1 GU/g to about 10 GU/g , and the glucose isomerase is used in an amount of from about 1 IGIU/g to about 100 IGIU/g.

16. A method as claimed in Claim 10 wherein the enzymatic treatment with the glucoamylase and glucose isomerase takes place at a temperature of from about 68° F (20° C) to about 176° F (80° C) and at a pH of from about 5 to about 8.

17. A method as claimed in Claim 13 wherein the soaking is at a temperature of from about 68° F (20° C) to about 212° F (100° C) and a pH of from about 5 to 8.

18. A method as claimed in Claim 12 wherein the cooking is at a temperature of from about 176° F (80° C) to about 212° F (100° C) and a pH of from about 5 to about 8, and the enzymatic treatment with the glucoamylase and glucose isomerase takes place at a temperature of from about 68° F (20° C) to about 176° F (80° C) and at a pH of from about 5 to about 8.

19. A method as claimed in Claim 12 wherein the enzymatic treatment with the glucoamylase and glucose isomerase takes place at a temperature of from about 131° F (55° C) to about 158° F (70° C) and a pH of from about 6 to 7.

20. A method as claimed in Claim 12 wherein the reducing sugar content of the cereal product ranges from about 10% by weight to about 25% by weight of total dry solids, and the amount of fructose is from about 5 percent by weight to about 45 percent by weight, based upon the total monosaccharide content of the cereal product.

21. A method as claimed in Claim 1 wherein the enzymatic treatment comprises subjecting the cooked cereal grain or grain fraction to alpha-amylase, glucoamylase and glucose isomerase to produce fructose.

22. A method as claimed in Claim 21 wherein the cereal grain or grain fraction is first subjected to alpha-amylase and then to glucoamylase and glucose isomerase.

23. A method as claimed in Claim 22 wherein the cereal grain or grain fraction is subjected to alpha-amylase during cooking, additional alpha-amylase is added after cooking and then the glucoamylase and glucose isomerase are added to produce fructose.

24. A method as claimed in Claim 23 wherein the enzymatic treatment with the glucoamylase and glucose isomerase takes place at a temperature of from about 68° F (20° C) to about 176° F (80° C) and at a pH of from about 5 to about 8.

25. A method as claimed in Claim 23 wherein the glucoamylase and glucose isomerase are added sequentially, the enzymatic treatment with the glucoamylase takes place at a temperature of from about 68° F (20° C) to 176° F (80° C), and a pH of from about 3 to about 8, and the enzymatic treatment with the glucose isomerase takes place at a temperature of from about 68.F (20° C) to about 212° F (100° C) and at a pH of from about 5 to about 9.

26. A method as claimed in Claim 22 wherein whole cereal grains are cooked in step (a) and the cooked grains retain discreteness during the enzymatic treatment with alpha-amylase, glucoamylase, and glucose isomerase.

27. A method as claimed in Claim 26 wherein the whole cereal grains are malted.

28. A method as claimed in Claim 26 wherein the discrete enzymatically treated cereal grains are formed by shredding, and the enzymes are inactivated by baking the shreds.

29. A method as claimed in Claim 4 wherein the enzymes are inactivated by toasting the flakes.

30. An enzyme-saccharified cereal product obtained by the process of Claim 1.

31. An enzyme-saccharified cereal product obtained by the process of Claim 1 which is a ready-to-eat cereal.

25

32. An enzyme-saccharified cereal product obtained by the process of Claim 1 which is a ready-to-eat shredded cereal biscuit.

33. An enzyme-saccharified cereal product as claimed in claim 31 wherein the reducing sugar content of the cereal product ranges from about 10% by weight to about 25% by weight of total dry solids, and the amount of fructose is from about 5 percent by weight to about 45 percent by weight, based upon the total monosaccharide content of the cereal product, the fructose content being at least about 1% by weight based upon the total dry solids of the cereal product.

34. A method for producing an enzyme-saccharified ready-to-eat cereal, comprising:

a) cooking whole cereal grains;

b) treating the cooked cereal grains with water and enzymes comprising glucose isomerase to produce a sweetening amount of fructose while retaining the cooked cereal grains in discrete form;

c) forming the enzyme treated cereal grain; and

d) inactivating the enzymes, the fructose content of the cereal product being at least about 1% by weight of the total dry solids of the cereal product.

35. A method as claimed in Claim 34 wherein the inactivation of the enzymes comprises heating the formed cereal grain.

36. A method as claimed in Claim 35 wherein the discrete enzymatically treated cereal grains are formed by shredding.

37. A method as claimed in Claim 36 wherein the enzymatically treated cereal grains are shredded into integral net-like sheets, the sheets are laminated, the laminate is cut, and the cut laminate is baked to inactivate the enzymes.

38. A method as claimed in Claim 35 wherein the enzymatic treatment comprises cooking the cereal grain in the presence of alpha-amylase to convert a portion of the cereal starch to dextrins, and then subjecting the cooked product to glucoamylase and glucose isomerase to produce fructose.

39. A method as claimed in Claim 38 wherein the alpha-amylase is used in an amount of from about 1 liq/g to about 1,000 liq/g., glucoamylase is used in an amount of from about 0.1 GU/g to about 10 GU/g , and the glucose isomerase is used in an amount of from about 1 IGIU/g to about 100 IGIU/g, the cooking is at a temperature of from about 176° F (80° C) to about 212° F (100° C) and a pH of from about 5 to about 8, and the enzymatic treatment with glucoamylase and glucose isomerase takes place at a temperature of from about 68° F (20° C) to about 176° F (80° C) and at a pH of from about 5 to about 8.

40. A method as claimed in Claim 39 wherein the amount of fructose is from about 5 percent by weight to about 45 percent by weight, based upon the total mcnosaccharide content of the cereal product.

41. An enzyme saccharified cereal product obtained by the process of Claim 37 which is a ready-to-eat shredded cereal biscuit.

42. An enzyme-saccharified cereal product obtained by the process of Claim 38 which is a ready-to-eat cereal flake.

43. A method for producing an enzyme-saccharified ready-to-eat cereal, comprising:

a) cooking a cereal grain fraction;

b) treating the cooked grain fraction with water and enzymes comprising glucose isomerase to convert a portion of the cereal starch to a sweetening amount of fructose;

c) forming the enzyme treated cereal grain fraction; and

d) inactivating the enzymes, the fructose content of the cereal product being at least about 1% by weight of the total dry solids of the cereal product.

44. A method as claimed in Claim 43 wherein the cereal grain fraction is wheat bran flour.

45. A method as claimed in Claim 44 wherein the enzymes comprise alpha-amylase, glucoamylase, and glucose isomerase.

46. A method as claimed in Claim 44 wherein the enzymatic treatment is at least partially prior to the cooking.

47. A method as claimed in Claim 44 wherein the enzyme treated cereal grain fraction is formed into pieces by extrusion.

48. A method as claimed in claim 44 wherein the starch content of the bran flour is from about 25% to about 45% by weight, on a dry basis.

49. A method as claimed in claim 1 wherein in step (a) the cereal grains or at least one cereal grain fraction comprises bran.

50. A process for producing a sweetened cereal product comprising:

a) moistening a cereal grain by adding an aqueous solution to a cereal grain containing cellulose and starch, in an amount sufficient to support conversion by enzymes of the cellulose and/or starch to fructose and in an amount such that substantially all of the solution is absorbed by the cereal;

b) disrupting the moistened cereal produced in step a) to an extent effective to promote the enzymatic conversion of the cellulose and/or starch to fructose by heating under pressure or treating with alkali;

c) adding to the disrupted, moistened cereal produced in step b) enzymes effective to convert cellulose and/or starch to fructose to produce a moistened cereal and enzyme mixture;

d) incubating said moistened cereal and enzyme mixture for a sufficient period of time to convert the cellulose and/or starch in said cereal to fructose at a temperature at which the enzymes are active; and

e) collecting a cereal product enriched in fructose.

51. The process of claim 50 wherein the enzymes are selected from the group consisting of cellulases, glucose isomerases, alpha-amylases and amylo-glucosidases.

52. The process of claim 51 wherein the enzymes comprise cellulase and glucose isomerase and the cellulase enzyme is added to the disrupted, moistened cereal before the glucose isomerase.

53. The process of claim 51 wherein the enzymes are cellulase, alpha-amylase and amylo-glucosidase and glucose isomerase and said cellulase and alpha-amylase and amylo-glucosidase enzymes are added to the disrupted, moistened cereal before glucose isomerase.

54. The process of claim 50 further including a drying step after said step of collecting.

55. The process of claim 54 wherein said drying step is carried out at a temperature in the range of between about 250° and 450° F.

56. The process of claim 50 wherein said step of incubating is carried out at a temperature of between about 25° and 80° C.

57. The process of claim 50 wherein said step of moistening and said step of adding the enzymes are carried out concurrently.

58. The process of claim 50 wherein said moistened cereal has a moisture content of about 40% to 75% weight/volume.

59. A sweetened cereal product enriched in fructose content produced by the process of claim 50.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 231 729 ( BERGKUIST et al.) * Claims 1-6,8,10,11,12; examples 6,7; page 12, lines 27-29; page 13, lines 16-18; page 14, lines 3-6; page 10, lines 14-17 * | 1,9,10-22,30, 42-45 | A 23 L 1/105 A 23 L 1/30 C 13 K 11/00 C 12 P 19/24 |
| Y | | 1-59 | |
| X,Y | EP-A-0 124 257 (GENERAL FOODS) * Claims 1,2-16; page 6, lines 18-31; page 7, lines 9-35; page 9, lines 13-18; page 10, line 26 - page 11, line 14 * | 1-59 | |
| A | WO-A-8 605 953 (LEWIS et al.) * Claims 1,2,7,11,24-31; examples 1,A,C; page 13, lines 10-19; example 5 * | 1-59 | |
| A | FR-A-2 215 467 (NAARDEN INTERNATIONAL) * Claims 1,2,3,7,8,9,10,11; page 2, line 36 - page 3, line 13; page 4, lines 1-15; examples 2,3,4,5,6,7 * | 1,7,8,9 ,10,12, 15,16-26 | TECHNICAL FIELDS SEARCHED (Int. Cl.4) |
| A | US-A-3 922 201 (HEBEDA et al.) * Claims 1-6; examples 1,2 * | 1,7,8,9 ,10,12, 15,16-26 | A 23 L C 13 K |
| D,A | NATURE, vol. 185, 26th March 1960, pages 924-925; J. BARTFAY: "Glucose-isomerase in barley malt" * Whole article * | 1 | |
| P,A | US-A-4 710 386 (FULGER et al.) * Claims 1-26 * | 1-59 | |

The present search report has been drawn up for all claims

| Place of search | Date of completion of the search | Examiner |
|---|---|---|
| THE HAGUE | 31-01-1989 | VAN MOER A.M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)